(19) Europäisches Patentamt / European Patent Office / Office européen des brevets

(11) **EP 4 346 862 B1**

(12) **EUROPEAN PATENT SPECIFICATION**


(45) Date of publication and mention of the grant of the patent: **23.09.2026 Bulletin 2026/39**

(21) Application number: **22730751.9**

(22) Date of filing: **20.05.2022**

(51) International Patent Classification (IPC):
***A61K 36/23*** *(2006.01)* ***A61P 17/02*** *(2006.01)*
***A61K 9/00*** *(2006.01)* ***A61K 9/06*** *(2006.01)*
***A61K 9/08*** *(2006.01)* ***A61K 47/02*** *(2006.01)*
***A61K 47/10*** *(2017.01)*

(52) Cooperative Patent Classification (CPC):
(C-Sets available)
**A61P 17/02; A61K 9/0014; A61K 9/06; A61K 9/08; A61K 36/23; A61K 47/02; A61K 47/10** (Cont.)

(86) International application number:
**PCT/EP2022/063761**

(87) International publication number:
**WO 2022/248361 (01.12.2022 Gazette 2022/48)**


(54) **PHARMACEUTICAL COMPOSITION COMPRISING A CENTELLA ASIATICA EXTRACT AND MEGLUMINE**

PHARMAZEUTISCHE ZUSAMMENSETZUNG MIT EINEM EXTRAKT AUS CENTELLA ASIATICA UND MEGLUMIN

COMPOSITION PHARMACEUTIQUE COMPRENANT UN EXTRAIT DE CENTELLA ASIATICA ET DE LA MÉGLUMINE


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.05.2021 EP 21382475**

(43) Date of publication of application:
**10.04.2024 Bulletin 2024/15**



(73) Proprietor: **Almirall S.A.**
**08022 Barcelona (ES)**

(72) Inventors:
- **ILLERA FONTANET, Miquel**
  **08022 Barcelona (ES)**
- **BOU ESPIGARES, Silvia**
  **08022 Barcelona (ES)**
- **DEL BARRIO FRANCO, Pedro Antonio**
  **08022 Barcelona (ES)**
- **ORTIZ FERRÓN, Francisco Javier**
  **08022 Barcelona (ES)**
- **HUGHES, Melanie Jane**
  **08022 Barcelona (ES)**
- **MACNAUGHTON, Katherine Jane**
  **08022 Barcelona (ES)**
- **MEARS, John James**
  **08022 Barcelona (ES)**

(74) Representative: **J A Kemp LLP**
**80 Turnmill Street**
**London EC1M 5QU (GB)**



(56) References cited:
**WO-A1-2016/181342** **WO-A1-2018/221487**
**WO-A1-2021/010953** **US-A1- 2019 282 538**

- **FANG M ET AL: "Solution formulation comprises Centella asiatica active ingredients, isotonic agent and solvent", WPI / 2017 CLARIVATE ANALYTICS,, vol. 2020, no. 62, 7 July 2020 (2020-07-07), XP002804773**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHANG L ET AL: "Moisturizing and repairing plant extract composition comprises Camellia seed extract, Aloe vera extract, oat extract, jojoba seed extract, Centella asiatica extract, grape seed extract, enzyme powder and Lactobacillus plantarum powder", WPI / 2017 CLARIVATE ANALYTICS,, vol. 2016, no. 76, 31 August 2016 (2016-08-31), XP002804772**
- **SUZUKI S ET AL: "Aqueous composition used for cosmetics e.g. pack cosmetics, comprises aqueous solution containing hydroxyethyl cellulose, methylcellulose and/or hypromellose, and moisture retaining component", WPI / 2017 CLARIVATE ANALYTICS,, vol. 2010, no. 47, 1 July 2010 (2010-07-01), XP002804775**
- **HE B ET AL: "Moisturizing dressing used in skin care for epidermis repair comprises sodium hyaluronate, sodium polystyrene sulfonate, xanthan gum, sodium polyacrylate, Carbomer, hydrolyzed glycosaminoglycans, Lubrajel oil, Centella and allantoin", WPI / 2017 CLARIVATE ANALYTICS,, vol. 2018, no. 79, 25 September 2018 (2018-09-25), XP002804774**
- **DATABASE WPI Week 201676, Derwent World Patents Index; AN 2016-592437, XP002804772, "Moisturizing and repairing plant extract composition comprises Camellia seed extract, Aloe vera extract, oat extract, jojoba seed extract, Centella asiatica extract, grape seed extract, enzyme powder and Lactobacillus plantarum powder"**
- **DATABASE WPI Week 202006, Derwent World Patents Index; AN 2020-65109B, XP002804773, "Solution formulation comprises Centella asiatica active ingredients, isotonic agent and solvent"**
- **DATABASE WPI Week 201879, Derwent World Patents Index; AN 2018-78501V, XP002804774, "Moisturizing dressing used in skin care for epidermis repair comprises sodium hyaluronate, sodium polystyrene sulfonate, xanthan gum, sodium polyacrylate, Carbomer, hydrolyzed glycosaminoglycans, Lubrajel oil, Centella and allantoin"**
- **DATABASE WPI Week 201047, Derwent World Patents Index; AN 2010-H57200, XP002804775, "Aqueous composition used for cosmetics e.g. pack cosmetics, comprises aqueous solution containing hydroxyethyl cellulose, methylcellulose and/or hypromellose, and moisture retaining component"**

Remarks:

The file contains technical information submitted after the application was filed and not included in this specification

(52) Cooperative Patent Classification (CPC): (Cont.)

C-Sets
**A61K 36/23, A61K 2300/00**

## Description

### Field of the Invention

The present invention relates to a pharmaceutical composition suitable for topical application which comprises *Centella asiatica* Extract.

### Background to the Invention

*Centella asiatica* is a perennial flowering herb from the family Apiaceae which is indigenous to the Indian subcontinent, Southeast Asia, and wetland regions of the Southeastern US. It is commonly known as Indian pennywort or Asiatic pennywort, but has a variety of other alternatively used names including Centella, Goyu Cola, Goyu Kola, Gotu Kola, marsh pennywort, pennyweed, sheeprot and spadeleaf. *Centella asiatica* is known to have medicinal properties and contains various active ingredients such as triterpenoids which include asiaticoside, asiatic acid, madecassic acid and madecassoside. It has been used in traditional medicine to treat various disorders, such as skin diseases and chronic inflammatory diseases and also in the treatment of wounds.

*Centella asiatica* extract has been formulated with different components. For example, Blastoestimulina ® by Almirall is a topical formulation which comprises Centella Asiatica extract (ECA) together with neomycin sulphate which is present for its antibiotic action. It has been used for the effective treatment of wounds.

CN 105 902 476 describes a composition comprising plant extracts including *Centella asiatica* extract that is used to repair the skin. WO 2016/181342 describes an ophthalmic composition comprising sodium hyaluronate and a pH adjuster, which may further comprise a hydrolat obtained from *Centella asiatica.* CN 111 374 941 describes a solution formulation comprising *Centella asiatica* for inhalation. CN 108 567 638 describes moisturizing dressings with epidermal repair function, which include *Centella asiatica.* WO 2021/010953 describes a topical skincare composition comprising a *Centella asiatica* extract in combination with a *Vincetoxicum officinale* extract and/or a *Populas alba* extract. US 2019/282538 describes alternative topical pharmaceutical compositions for treating dermatological conditions, which comprise ivermectin. WO 2018/221487 describes an injectable composition containing eribulin, or a pharmaceutically acceptable salt thereof, and a pH modifier. JP 2010 143868 describes an aqueous composition comprising hydroxyethyl cellulose, methylcellulose and/or hypromellose, and a moisturizing component.

### Summary of the Invention

The present invention is directed to new pharmaceutical compositions comprising *Centella Asiatica* extract (ECA). Blastoestimulina ® is a very effective formulation for the treatment of wounds. However, it would be advantageous to provide a wound healing formulation which did not include neomycin sulfate in light of the drive to reduce antibiotic consumption, in particular due to the effects of antibiotic resistance.

It was found that removal of neomycin sulphate from a formulation comprising ECA resulted in a product having reduced stability in comparison to the original formulation. The present inventors have investigated and developed new compositions comprising ECA which have improved levels of stability and can therefore show greater effectiveness when used in the treatment of wounds.

Accordingly, the present invention provides a pharmaceutical composition suitable for topical application which comprises *Centella Asiatica* extract (ECA) and a pH modifying agent which is meglumine, and wherein said composition has a pH from 9.0 to 10.5.

It is preferred that the composition comprises from 0.25 to 5.0 wt% ECA. In particular, it is preferred that the composition comprises from 0.5 to 2.5 wt% ECA.

Preferably, the composition comprises a carrier or vehicle selected from water, petroleum hydrocarbons, fatty acids, fatty acid esters, fatty alcohols, fatty alcohol ethers, fatty alcohol esters, $C_2$-$C_8$ linear or branched, saturated or unsaturated alcohols, polyols, aromatic alcohols, alkyleneglycol ethers, alkyleneglycol esters, natural waxes, silicones or mixtures thereof.

It is particularly preferred that the carrier or vehicle is selected from water, fatty acid esters, polyols or mixtures thereof.

Preferably, the composition further comprises one or more additives selected from anti-irritants agents, anti-oxidants agents, chelating agents, emollients, penetration enhancing agents, preservative agents, solubilizing agents, thickening agents, wetting agents, fragrances or mixtures thereof.

It is also preferred that the composition is formulated in the form of a cream, a gel, an ointment, a paste, a suspension, a lotion, a foam, a spray, an aerosol or a solution. It is particularly preferred that composition is formulated in the form of an ointment.

Preferably, the composition comprises:

(a) from 0.25 to 5.0 wt% ECA
(b) from 0.2 to 2.5 wt% pH modifying agent (meglumine)
(c) from 0.5 to 2.5 wt% additives
(d) from 90 to 98.8 wt % carrier or vehicle.

It is further preferred that the composition comprises:

(a) from 0.8 to 1.2 wt% ECA
(b) from 0.5 to 2 wt% meglumine
(c) from 0.8 to 1.2 wt% of a perfume or mixture of perfumes
(d) from 15 to 25 wt % diethylene glycol monostearate
(e) from 25 to 35 wt% propylene glycol
(f) from 5 to 15 wt% corn oil
(g) from 25 to 50 wt% water

Also provided is a composition of the invention for use in the treatment of wounds (such as fissures, ulcers, sores, pressure ulcers or burns).

**Detailed Description of the Invention**

Any references herein to a method of treatment of the human or animal body using a composition of the invention should be interpreted as meaning said composition for use in such a method.

As used herein, the term "*Centella Asiatica* extract", "ECA" or "extract" refers to material extracted from the plant *Centella Asiatica.* The entire plant can be used for the extraction process or the extraction process can be performed on any part of the plant including the flowers, leaves, stems, roots, seeds, fruit and mixtures thereof. Extracts may be obtained using any suitable method known in the art including milling, grinding, maceration, infusion, percolation and decoction, Soxhlet extraction, microwave assisted extraction, ultrasound-assisted extraction, sonication extraction, solvent extraction, accelerated solvent extraction, and supercritical fluid extraction. Suitable extraction solvents may include water, ketones, esters, $C_1$ to $C_6$ alcohols, hydrocarbons and mixtures thereof.

The compositions of the invention have a pH from 9.0 to 10.5, preferably from 9.3 to 10.2 and more preferably from 9.5 to 10.0.

In a preferred embodiment, the pH of the composition is from 9.4 to 10.0 and the pH changes by less than ± 0.3 when stored at 25°C for 28 days. It is further preferred that the pH changes by less than ± 0.3 when stored at 40°C for 28 days.

In another preferred embodiment, the pH of the composition is from 9.4 to 10.0 and the pH changes by less than ± 0.5 when stored at 25°C for 3 months. It is further preferred that the pH changes by less than ± 0.8 when stored at 40°C for 3 months.

The pH modifying agent is an organic pH modifying agent which is meglumine. The present inventors have determined that maintaining the pH of the composition within the desired range assists in providing a composition which has a high level of stability. The pH modifying agent has an effect on the stability of the composition and the use of such an agent provides a composition with an enhanced level of stability.

In one embodiment, the pH modifying agent is meglumine and the pH of the composition is from 9.3 to 9.8.

Typically, the carrier or vehicle is selected from water, petroleum hydrocarbons, fatty acids, fatty acid esters, fatty alcohols, fatty alcohol ethers, fatty alcohol esters, $C_2$-$C_8$ linear or branched, saturated or unsaturated alcohols, polyols, aromatic alcohols, alkyleneglycol ethers, alkyleneglycol esters, natural waxes and silicones or mixtures thereof.

Preferably, the carrier or vehicle is selected from water, fatty acid esters, polyols or mixtures thereof.

The topical pharmaceutical compositions according to the invention may optionally further comprise other well-known pharmaceutically and/or cosmetically acceptable additives, such as, e.g. anti-irritants, antioxidants, chelating agents, emollients, penetration enhancing agents, preservative agents, solubilizing agents, thickening agents, wetting agents, fragrances and the like, or mixtures thereof.

Suitable petroleum hydrocarbons , i.e. mineral oils, paraffins and waxes from petroleum are: hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated $C_{18}$ -$C_{30}$ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffines waxes (which consist of $C_{40}$-$C_{55}$ compounds which contain, in addition to normal hydrocarbons, large amounts of iso-alkanes and naphtenes with long alkyl side-chains, the iso-alkanes forming microcrystals, the microcrystalline paraffines waxes having

mean molecular weights comprised between 500 and 800 g/mol, being solids at room temperature, and having melting points comprised between 60°C and 90°C), or mixtures thereof. Preferred petroleum hydrocarbons are hard paraffin, liquid paraffin, light liquid paraffin, white soft paraffin or mixture thereof, being particularly preferred liquid paraffin, white soft paraffin or mixtures thereof.

Suitable fatty acids are $C_6$ -$C_{24}$ carboxylic acids, saturated or unsaturated, purified or synthetic, from vegetable and animal fats and oils, such as 2-ethylhexanoic acid, arachidic acid, arachidonic acid, behenic acid, capric acid, caproic acid, caprylic acid, castor oil acid, coconut acid, corn acid, cottonseed acid, elaidic acid, erucic acid, gadoleic acid, isostearic acid, lauric acid, linoleic acid, linolenic acid, linseed acid, myristic acid, oleic acid, olein, olive acid, olive pomace, palm acid, palm kernel acid, palmitic acid (cetylic acid), palmitoleic acid, peanut acid, pelargonic acid, petroselinic acid, rapeseed acid, rice bran acid, ricinoleic acid, safflower acid, soy acid, stearic acid, sunflower seed acid, tall oil acid, tallow acid, undecanoic acid, undecylenic acid, wheat germ acid or mixtures thereof.

Fatty acid esters as used herein represent the covalent compounds formed between the fatty acids as described above and any suitable alcohol. Suitable fatty acid esters according to the present invention are preferably selected from (i) fats and oils, (ii) alkyl fatty esters, (iii) alkoxylated fatty acid esters and (iv) sorbitan fatty acid esters. (i) Fats and oils are the glyceryl esters of fatty acids (triglycerides) normally found in animal and plant tissues, including those which have been hydrogenated to reduce or eliminate unsaturation. Also included are synthetically-prepared esters of glycerin and fatty acids (mono-, di-, and triglycerides). The fatty acids esterifying the different positions of glycerin can be different, giving rise to a large amount of possible combinations, including positional combinations. The position of the different fatty acids in natural triglycerides is not random, but rather it depends on the origin of the fat. The triglycerides which are more simple are those constituted by a sole fatty acid. Glyceryl esters of fatty acids can be advantageously chosen, for example, from the group consisting of synthetic, semisynthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by- products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like. Other examples of glyceryl esters are glyceryl monobehenate, glyceryl dibehenate, glyceryl monooleate, glyceryl dioleate, glyceryl monostearate, glyceryl distearate, glyceryl monopalmitosteareate and glyceryl dipalmitosteareate. (ii) Alkyl fatty esters represent esters of fatty acids as described above and linear or branched, saturated or unsaturated $C_1$-$C_{30}$ alcohols, ethylene glycol or propylene glycol. These fatty acid esters can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2- ethylhexyl cocoate, 2-hexyldecyl stearate, 2- ethylhexyl isostearate, 2-octyldodecyl palmitate, cetyl palmitate, oleyl oleate, oleyl erucate, erucyl oleate, erucyl erucate, propyleneglycol monocaprylate, propyleneglycol dicaprylate, propyleneglycol monopalmitostearate, propyleneglycol monostearate, propyleneglycol alginate, ethyleneglycol monopalmitostearate, ethyleneglycol monostearate, as well as synthetic, semisynthetic and natural mixtures of such esters, such as jojoba oil (a natural mixture of esters of monounsaturated monocarboxylic acids with a $C_{18}$-$C_{24}$ chain with also monounsaturated monoalcohols and with a long $C_{18}$-$C_{24}$ chain). (iii) Alkoxylated fatty acid esters are formed when a fatty acid is reacted with an alkylene oxide or with a preformed polymeric ether. The resulting product may be a monoester or a diester or a mixture of the two, depending on the reaction conditions. Typical representatives include PEG-6 isosteareate, PEG-2 stearate, PEG-4 stearate, PEG-8 stearate, PEG-12 stearate, PEG-20 stearate, PEG-40 stearate, PEG-50 stearate, PEG- 100 stearate and PPG-17 dioleate. (iv) Sorbitan fatty acid esters are the reaction product of the esterification of sorbitan an one or more fatty acids as defined above. Examples of suitable sorbitan fatty acid esters include sorbitan monolaurate, sorbitan monooleate, sorbitan monopalmitate, sorbitan monostearate, sorbitan sesquioleate, sorbitan trioleate and sorbitan tristearate, and polysorbates such as polysorbate 20, polysorbate 40, polysorbate 60, polysorbate 65, polysorbate 80 or polysorbate 85.

It is preferred to use vegetable oils such as avocado oil, almond oil, hazelnut oil, babassu palm oil, coconut oil, rapeseed oil, wheat germ oil, sunflower oil, linseed oil, macadamia nut oil, corn oil, walnut oil and olive oil. It is particularly preferred to use corn oil.

It is preferred to use alkoxylated fatty acid esters such as PEG-6 isosteareate, PEG-2 stearate, PEG-4 stearate, PEG-8 stearate and PEG-12 stearate. It is particularly preferred to use PEG-2 stearate.

Suitable fatty alcohols according to the present invention are $C_6$-$C_{24}$ alcohols from vegetable and animal fats and oils, such as 2-octyldodecanol, 2-ethylhexanoyl alcohol, arachidyl alcohol, behenyl alcohol, caprylic alcohol, caproyl alcohol, capric alcohol, castor oil alcohol, ceterayl alcohol, palmityl (cetyl) alcohol, coconut alcohol, cotton alcohol, decyl alcohol, elaidyl alcohol, erucyl alcohol, gadoleyl alcohol, isostearyl alcohol, lauryl alcohol, linoleyl alcohol, linseed alcohol, myristyl alcohol, olein alcohol, olive pomace alcohol, oleyl alcohol, olive alcohol, palm alcohol, palm kernel alcohol, palmitoyl alcohol, petroselinic alcohol, rapeseed alcohol, ricinoleyl alcohol, safflower alcohol, soy alcohol, stearyl alcohol,

sunflower alcohol, tall oil alcohol, tallow alcohol, tridecyl alcohol, or technical grade mixtures thereof such as cetostearyl alcohol.

Suitable fatty alcohol ethers according to the present invention are ethers formed from the reaction of a fatty alcohol as defined above with an alkylene oxide, generally ethlyene oxide or propylene oxide. Fatty alcohols ethers can be advantageously selected from the group consisting of ceteth-20, isosteareth-10, myreth-10, laureth-16, oleth-16, polyoxyl 6 cetostearyl ether, polyoxyl 20 cetostearyl ether, polyoxyl 25 cetostearyl ether, polyoxyl 2 cetyl ether, polyoxyl 10 cetyl ether, polyoxyl 20 cetyl ether, polyoxyl 4 lauryl ether, polyoxyl 9 lauryl ether, polyoxyl 23 lauryl ether, polyoxyl 2 oleyl ether, polyoxyl 10 oleyl ether, polyoxyl 20 oleyl ether, polyoxyl 2 stearyl ether, polyoxyl 10 stearyl ether, polyoxyl 21 stearyl ether or polyoxyl 100 stearyl ether. Suitable fatty alcohol esters are the product of the reaction of a fatty alcohol as defined above and a linear of branched, saturated or unsaturated $C_1$-$C_5$ carboxylic acid. Examples of fatty alcohol esters are lauryl acetate, myristyl acetate, cetyl acetate, stearyl acetate and stearyl propionate.

Suitable aromatic alcohols according to the present invention are preferably selected from (i) arylalkanols, (ii) aryloxyalkanols (glycol monoaryl ethers) and (iii) oligoalkanol aryl ethers. The (i) arylalkanols used according to the invention have the formula Ar-$(CHR)_n$-OH where R independently represents H or $C_1$-$C_6$ alkyl, with n being an integer, and preferably 1 to 10, more preferably 1 to 6, and in particular 1 , 2, 3 or 4. The group Ar can be a substituted or unsubstituted aryl group, for example phenyl or naphtyl. Example arylalkanols are benzyl alcohol, 3-phenylpropan-1-ol, phenethyl alcohol, veratryl alcohol (3,4-dimethoxyphenylmethyl alcohol) and 2-methyl-1-phenyl-2- propanol. The (ii) aryloxyalkanols used according to the invention have the formula Ar-O-$(CHR)_n$-OH where R independently represents H or $C_1$-$C_6$ alkyl, with n being an integer, and preferably 2 to 10, more preferably 2 to 6, and in particular 2 or 3. The group Ar can be a substituted or unsubstituted aryl group, for example phenyl or naphtyl. Example arlyoxyalkanols used according to the invention are phenoxyethanol, 1-phenoxypropan-2-ol, 2-phenoxylpropan-1-ol, 3-phenoxypropan-1-ol, or mixtures thereof. The (iii) oligoalkanol aryl ethers include, for example, phenoxy diethanol, triethanol and oligoethanol, and phenoxy dipropanol, tripropanol and oligopropanol.

Suitable polyols according to the present invention are preferably water-soluble polyols such as polyhydric alcohols with two or more hydroxyl groups in their molecule. Specific examples can include ethylene glycol, propylene glycol, 1,3-butylene glycol, 1,4-butylene glycol, hexylene glycol, dipropylene glycol, glucose, fructose, galactose, mannose, ribose, erythrose, maltose, maltitose, maltotriose, sucrose, xylitol, sorbitol, threitol, erythritol, glycerol, polyglycerol and starch alcohols. Preferred polyols are ethylene glycol, propylene glycol, 1,3-butylene glycol, 1,4-butylene glycol, dipropylene glycol, hexylene glycol, glycerol, polyglycerol, and mixtures thereof. Preferred polyols include ethylene glycol, propylene glycol, 1,3-butylene glycol, 1,4-butylene glycol, hexylene glycol, dipropylene glycol. Particularly preferred polyols include propylene glycol.

Suitable alkyleneglycol esters are esters of ethylene glycol or propylene glycol with the $C_6$-$C_{24}$ fatty acids defined above. Alkyleneglycol esters can be advantageously selected from the group consisting of ethylene glycol monopalmitostearate, ethylene glycol monostearate, propylene glycol monocaprylate, propylene glycol dicaprylate, propylene glycol dicaprylocaprate, propylene glycol monopalmitostearate, propylene glycol monostearate or propylene glycol alginate. Suitable alkylene glycol ethers are polymers of ethylene oxide (polyethylene glycol monomethyl ether) or propylene oxide (polypropylene glycol monomethyl ether).

Alkyleneglycol ethers can be advantageously selected from the group consisting of PEG 200, PEG 400, PEG 540, PEG 600, PEG 900, PEG 1000, PEG 1450, PEG 1540, PEG 2000, PEG 3000, PEG 3350, PEG 4000, PEG 4600, PEG 8000, PPG-9, PPG-10, PPG-17, PPG-20, PPG-26, PPG-30 or PPG-55.

Suitable natural waxes are the candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

Silicones suitable according to the present invention are cyclic and/or linear silicones, which can be found as monomers generally characterized by structural elements such as:

$$R_2{-}O{-}\underset{\displaystyle R_4}{\overset{\displaystyle R_1}{Si}}{-}O{-}R_3$$

where the silicon atoms can be substituted by alkyl or aryl radicals equal or different, represented here generally by $R_1$-$R_4$ groups.

Linear silicones with siloxane units suitable according to the present invention are generally characterized by structural elements such as:

where the silicon atoms can be substituted by alkyl or aryl radicals equal or different, are represented here in general by $R_1$-$R_4$ groups (meaning the number of different radicals is not necessarily limited to 4), m can take values from 2 to 200,000.

Cyclic silicones suitable according to the present invention are generally characterized by structural elements such as:

R1
R2
O–Si–O–Si
R3
R4
n

where the silicon atoms can be substituted by alkyl or aryl radicals equal or different, represented here generally by $R_1$-$R_4$ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula $[(CH_3)_2SiO]_x$ in which x is 3 to 6, or short chain linear methyl siloxanes having the formula $(CH_3)_2SiO[(CH_3)_2SiO]_ySi(CH_3)_3$ in which y is 0 to 5.

Some suitable cyclic methyl siloxanes are hexamethylcyclotrisiloxanes (D3), a solid with a boiling point of 134°C and the formula $[(Me_2)SiO]_3$ ; octamethylcyclotetrasiloxane (D4) with a boiling point of 176°C, a viscosity of 2.3 $mm^2/s$, and the formula $[(Me_2)SiO]_4$; decamethylcyclopentasiloxane (D5) (cyclomethicone) with a boiling point of 210°C, a viscosity of 3.87 $mm^2/s$, and the formula $[(Me_2)SiO]_5$; and dodecamethylcyclohexasiloxane (D6) with a boiling point of 245°C, a viscosity of 6.62 $mm^2/s$ and the formula $[(Me_2)SiO]_6$ .

Some suitable short linear methyl siloxane are hexamethyldisiloxane (MM) with a boiling point of 100°C, viscosity of 0-65 $mm^2/s$, and formula $Me_3SiOMe_3$; octamethyltrisiloxane (MDM) with a boiling point of 152°C, viscosity of 1.04 $mm^2/s$, and formula $Me_3SiOMe_2$ $SiOSiMe_3$; decamethyltetrasiloxane (MD2M) with a boiling point of 194°C, viscosity of 1.53 $mm^2/s$, and formula $Me_3SiO(MeSiO)_2SiMe_3$; dodecamethylpentasiloxane (MD3M) with a boiling point of 229°C, viscosity of 2.06 $mm^2/s$, and formula $Me_3SiO(Me_2SiO)_3SiMe_3$; tetradecamethylhexasiloxane (MD4M) with a boiling point of 245°C, viscosity of 2.63 $mm^2/s$, and formula $Me_3SiO(Me_2SiO)_4SiMe_3$; and hexadecamethylheptasiloxane (MD5M) with a boiling point of 270°C, viscosity of 3.24 $mm^2/s$, and formula $Me_3SiO(Me_2SiO)_5SiMe_3$.

Furthermore, long chain linear siloxanes such as phenyltrimethicone, bis(phenylpropyl)dimethicone, dimethicone, dimethiconol, cyclomethicone, (octametilciclotetrasiloxane), hexamethylcyclotrisiloxane, poly(dimethylsiloxane), cetyldimethicone and behenoxy dimethicone are also included.

In addition, mixtures of cyclomethicone and isotridecyl isononanoate and of cyclomethicone and 2-ethylhexyl isostearate are also suitable silicones according to the invention.

It is preferred that the carrier or vehicle comprises a mixture of water, vegetable oil, alkoxylated fatty acid ester and polyol. It is further preferred to use a mixture of water, vegetable oil selected from avocado oil, almond oil, hazelnut oil, babassu palm oil, coconut oil, rapeseed oil, wheat germ oil, sunflower oil, linseed oil, macadamia nut oil, corn oil, walnut oil and olive oil, alkoxylated fatty acid ester selected from PEG-6 isosteareate, PEG-2 stearate, PEG-4 stearate, PEG-8 stearate and PEG-12 stearate and polyol selected from ethylene glycol, propylene glycol, 1,3-butylene glycol, 1,4-butylene glycol, hexylene glycol, dipropylene glycol. It is particularly preferred to use a mixture of water, corn oil, PEG-2 stearate and propylene glycol.

The topical pharmaceutical compositions according to the invention may optionally further comprise other well-

known pharmaceutically and/or cosmetically acceptable additives, such as, e.g. anti-irritants, antioxidants, buffering agents (pH adjusting agents), chelating agents, emollients, penetration enhancing agents, preservative agents, solubilizing agents, thickening agents, wetting agents, and the like, or mixtures thereof.

The composition can also comprise one or more additives selected from anti-irritants agents, antioxidants agents, chelating agents, emollients, penetration enhancing agents, preservative agents, solubilizing agents, thickening agents, wetting agents, fragrances or mixtures thereof.

Examples of suitable anti-irritants are aloe vera, chamomile, alpha-bisabolol, cola nitida extract, green tea extract, tea tree oil, licoric extract, batyl alcohol (a-octadecyl glyceryl ether), selachyl alcohol (α-9-octadecenyl glyceryl ether), chimyl alcohol (a-hexadecyl glyceryl ether), panthenol, allantoin, caffeine or other xanthines, glycyrrhizic acid and derivatives thereof, and mixtures thereof. Antioxidants used can be any antioxidants which are suitable or customary for cosmetic and/or dermatological applications.

Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. a-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteinesulphoximine, buthionine sulphones, penta-, hexa-, heptathionine sulphoximine) in very small tolerated doses (e.g. pmol to μηηol/kg), also (metal) chelating agents (e.g. a- hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, $ZnSO_4$ ), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Suitable emollients, which can be used in the composition of the present invention include, for example, dodecane, squalane, cholesterol, isohexadecane, isononyl isononanoate, PPG ethers, petrolatum, lanolin, safflower oil, castor oil, coconut oil,cottonseed oil, palm kernel oil, palm oil, peanut oil, soybean oil, polyol carboxylic acid esters, derivatives thereof, and the like, and combinations thereof.

Examples of suitable penetration enhancing agents can include, e.g., dimethylsulfoxide (DMSO), N-methyl pyrrolidine, dimethyl formamide (DMF), allantoin, urazole, N,N- dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzethonium chloride; and mixtures thereof. The amount of preservative generally utilized will vary depending upon the preservative selected.

Examples of solubilizing agents are, for example, nonionic surfactants from at least one of the following groups: products of the addition of 1 to 30 moles of ethylene oxide and/or 0 to 5 moles of propylene oxide onto linear $C_8$-$C_{22}$ fatty alcohols, $C_{12}$-$C_{22}$ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3

to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di- and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear $C_8$-$C_{22}$ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate, or sodium cetostearyl sulphate; or fatty acids and salts thereof, such as oleic acid, sodium oleate and triethanolamine oleate.

Fragrances include compounds which can be added to the composition to impart a pleasant aroma and include lemon oil, ionone, diphenyl oxide, ylang ylang oil, cedryl acetate, isoeugenol, cinnamic alcohol, aurantheol, methyl anthranilate, vanillin, bergamot oil, eugenol, cananga oil , citral, tetrahydro linalool, patchouli oil, methyl isoeugenol, hexylcinnamic aldehyde, sandalwood oil, geraniol, terpenyl acetate, benzyl isoeugenol, rhodinol, diphenyl methane, hydroxycitronellal, methyl benzoate, benzyl propionate, palmarose, orange perfume, geranium perfume, methyl gamma ionone and lavender perfume. It is particularly preferred to use a mixture of geranium perfume and lavender perfume.

The composition of the invention preferably comprises from 0.25 to 5.0 wt% ECA, more preferably comprises from 0.5 to 2.5 wt% ECA, and still more preferably comprises 0.75 to 1.5 wt% ECA. In a preferred embodiment, the composition comprises from 0.8 to 1.2 wt% ECA. In a particularly preferred embodiment, the composition comprises around 1 wt% ECA.

The amount of pH modifying agent used is mainly dependent upon ensuring that the composition has an appropriate overall pH. However, the composition preferably comprises from 0.2 to 2.5 wt% pH modifying agent, more preferably comprises from 0.25 to 2.0 wt% pH modifying agent, and still more preferably comprises 0.35 to 1.5 wt% pH modifying agent. The amount of such an agent which is used is preferably from 0.25 to 2.0 wt%, more preferably 0.5 to 1.5 wt% and particularly preferably 0.8 to 1.2 wt%.

The amount of additives used in the composition is preferably from 0.5 to 5.0 wt%, more preferably from 0.5 to 2.5 wt% and particularly preferably from 0.5 to 2.0 wt%.

The carrier or vehicle used generally makes up the balance of the composition. However, typically the amount of carrier or vehicle makes up to 80 to 99 wt% of the composition, preferably from 85 to 99 wt% or 90 to 99 wt% of the composition and more preferably from 95 to 98 wt % of the composition. In one embodiment, the carrier or vehicle comprises 90 to 99.05 wt% of the composition. In another embodiment, the carrier or vehicle comprises 93.5 to 98.75 wt% of the composition. In a further embodiment, the carrier or vehicle comprises 95.0 to 98.5 wt% of the composition.

The carrier or vehicle will generally comprise water as one of its components such that the water of the composition will preferably be 20 to 70 wt%, more preferably 25 to 60% and further more preferably 25 to 50 wt%.

In one embodiment, the composition of the invention comprises:

(a) from 0.25 to 5.0 wt% ECA
(b) from 0.2 to 2.5 wt% pH modifying agent (meglumine)
(c) from 0.5 to 5.0 wt% additives
(d) from 90 to 99.05 wt % carrier or vehicle.

In another embodiment, the composition of the invention comprises:

(a) from 0.5 to 2.5 wt% ECA
(b) from 0.25 to 2.0 wt% organic pH modifying agent (meglumine)
(c) from 0.5 to 2.5 wt% additives
(d) from 93.5 to 98.75 wt % carrier or vehicle selected from water, fatty acid esters, polyols or mixtures thereof.

In a further embodiment, the composition of the invention comprises:

(a) from 0.5 to 1.5 wt% ECA
(b) from 0.5 to 2.0 wt% organic pH modifying agent (meglumine)
(c) from 0.5 to 2.0 wt% fragrances
(d) from 95.0 to 98.5 wt % carrier or vehicle selected from water, fatty acid esters, polyols or mixtures thereof.

In a further preferred embodiment, the composition of the invention comprises:

(a) from 0.8 to 1.2 wt% ECA
(b) from 0.5 to 2 wt% meglumine
(c) from 0.8 to 1.2 wt% of perfume
(d) from 15 to 25 wt % diethylene glycol monostearate
(e) from 25 to 35 wt% propylene glycol
(f) from 5 to 15 wt% corn oil
(g) from 25 to 50 wt% water

In one embodiment, the compositions of the invention consist of the formulations provided above.

The composition of the invention can be formulated into the form of a cream, a gel, an ointment, a paste, a suspension, a lotion, a foam, a spray, an aerosol or a solution. Preferably, the composition is formulated into a cream or an ointment. More preferably, the composition is formulated into an ointment.

The viscosity of the composition of the invention will depend on the form of the composition. For instance, in the case of a cream, the viscosity is typically in the range of 2,000 to 15,000 mPa.s, preferably in the range of 2,500 to 10,000 mPa.s, more preferably in the range of 3,000 to 7,000 mPa.s measured at 20°C using a DIN-Rotations Rheometer (Paar Physica); Measuring System Z 3 DIN ; D= 57 1/s. In the case of a gel, the viscosity is typically in the range of 300 to 1 ,500 mPa.s, preferably in the range of 500 to 1 ,200 mPa.s, more preferably in the range of 600 to 900 mPa.s measured at 20°C using a DIN-Rotations Rheometer (Paar Physica); Measuring System Z 3 DIN; D= 57.2/s. In the case of an ointment, the viscosity is typically in the range of 15,000 to 45,000 mPa.s, preferably in the range of 20,000 to 40,000 mPa.s and more preferably in the range of 20,000 to 35,000 mPa.s.

The functions of the skin include protecting the body from external harm, sensing environmental changes, and maintaining physiological homeostasis. It is important that the integrity of the skin is maintained and that any wounds to the skin are appropriately healed. Effective wound healing involves skin regeneration and repair and is an important aspect of healthcare. Poor wound healing is a major public health issue worldwide and can lead to serious conditions such as infections and other problems which can in some circumstances become life threatening.

In one embodiment, the wound is a topical wound. In one embodiment, the wound comprises a cut, abrasion, diabetic wound, canker, ulcer, aphthous stomatitis, sore, burn, surgical wound, abrasion, and/or surgical adhesion. The composition of the invention is particularly effective when used in the treatment of wounds (such as fissures, ulcers, sores, pressure ulcers or burns), and symptoms of itch, redness, flaking, dryness and/or roughness of the skin, scalp and/or of the mucous membrane. Preferably, the composition of the invention is used in the treatment of wounds. It can also be used as an adjuvant in the healing of surgical wounds or skin graft attachments.

A wound can be caused by tissue damage due to, for example, chemical damage, thermal damage, bites, trauma, etc. In one embodiment, the wound is a chronic wound. In one embodiment, the wound is an acute wound.

The composition of the invention can be used in the treatment of wounds.

The invention also relates to the composition of the invention for use in a method for treating a wound on a subject which comprises applying to the wound an effective amount of the composition.

The method of using the composition of the invention can involve applying it to completely cover the affected area, forming an occlusive barrier. The usual frequency of application is one to four times daily, preferably, one to three times daily and more preferable once or twice daily. As used herein, the term " effective amount" refers to an amount sufficient to effect treatment when administered to a patient in need of treatment.

The following examples are given in order to illustrate the present invention, but should not be considered as limiting of the essential aspects of its subject, as set out in the preceding portions of this description.

## Examples

Any examples falling outside the scope of the claims are included for reference purposes only.

### Example 1 - testing buffer solutions

ECA stock solution preparation

First, 33.3 mg/mg ECA stock solution was prepared in a 100g batch size by dissolving the required amount of ECA in the required quantity of propylene glycol at 70°C. The solution was allowed to cool to room temperature once the ECA was solubilized.

100 mL each the following stock solutions were prepared by making to the mark with deionised water after dissolving the specified solids by repeated inversion or by using a flask shaker:

i. 1% w/v meglumine: 1.0g of Meglumine
ii. 0.01M NaOH: 0.04g of sodium hydroxide pellets
iii. 1.0M NaOH: 4.0g of sodium hydroxide pellets
iv. 0.1M triethanolamine: 1.5g of triethanolamine
v. 0.1M sodium carbonate: 1.1g sodium carbonate
vi. 0.1M sodium bicarbonate: 0.84g sodium bicarbonate
vii. 1.0M HCl: Add ~50 mL of deionised water into a 100 ml volumetric flask, followed by 8.2 mL 37% hydrochloric acid. Gently swirl the volumetric flask, then make to the mark with the deionised water and invert the flask several times to mix.

The carbonate buffer, pH 10.1, was prepared from its two stock solutions as follows:

(a) 70 mL of the sodium carbonate stock solution was added to a 100 mL beaker.
(b) approximately 25 mL of the sodium bicarbonate stock solution was added to the beaker and the solution stirred with a magnetic flea.
(c) If the pH is not within the target of 9.6 to 10.4, the pH was adjusted dropwise by addition of 0.1 M $Na_2CO_3$ or 0.1M $NaHCO_3$ - the mass of 0.1M $Na_2CO_3$ or 0.1 M $NaHCO_3$ added and the final pH were recorded.
(d) The solution was transferred to a 100 mL volumetric flask and made to volume with deionised water.
(e) The flask was inverted several times to mix.

The glycine-sodium hydroxide buffer, pH 10.0, was prepared as follows:

(a) 0.375g of Glycine was added to 80 mL of deionised water in a 250 mL beaker and stirred with a magnetic flea until the contents is completely homogeneous.
(b) 0.128g of sodium hydroxide pellets was added to the beaker and stirring continued until completely dissolved.
(c) If the pH is not within the target of 10.0, the pH was adjusted by dropwise addition of either 1M HCl or 1M NaOH solution -the mass of 1M HCl/1M NaOH solution added and the final pH were recorded.
(d) The solution was transferred to a 100 mL volumetric flask and made to volume with deionised water.
(e) The flask was inverted several times to mix.

From each buffer-stock solution, a 5 mL sample was transferred into separate 8 mL glass vials. These samples were blanks to be used in the testing.

The required amount of the 3.33% w/w ECA stock solution in propylene glycol was added to the buffers to achieve an API concentration of 1% w/w. The pH of each solutions was then adjusted to the target pH and made to final weight (20g). The composition of each of the tested solutions is shown in Table 1.

The compositions were subjected to stability testing to investigate their stability over time at different temperatures. The stability of the ECA-containing solutions were investigated through assay and by monitoring the physical stability (appearance) and pH of the solutions. The assay results are shown in Table 2 and the physical stability and pH results in Table 3.

The assay was performed using the following protocol:
1.5g of the composition of the solution was weighed into a 25 ml volumetric flask. 15 ml of methanol was added to the volumetric flask and the mixture vortex mixed form 30 seconds and then stirred on an orbital shaker for 30 minutes or until dissolved. The flask was made to the volume with methanol followed by mixing well by inverting 15 times. Centrifugation was then carried out (4500 rpm for 20 minutes) and the supernatant filtered through a 0.22 μm nylon filter. 1ml of the filtered

sample was then transferred into a HPLC vial and analysed by HPLC.

| Tested solution | Ia | Ib | IIa | IIb | IIIa | IIIb | IVa | IVb | Va | Vb |
|---|---|---|---|---|---|---|---|---|---|---|
| ECA/PG solution | 30% | 30% | 30% | 30% | 30% | 30% | 30% | 30% | 30% | 30% |
| 0.01 M NaOH solution | 65% | 65% | | | | | | | | |
| 1.0 M HCl | q.s (pH 10.5) | q.s (pH 10.5) | | | | | | | | |
| Glycine-sodium Hydroxide buffer | | | 65% | 65% | | | | | | |
| 1.0 M NaOH solution | | | q.s (pH 10.5) | q.s (pH 10.5) | | | | | | |
| 1.0 M HCl | | | q.s (pH 10.5) | q.s (pH 10) | q.s (pH 10.5) | q.s (pH 10) | q.s (pH 10.5) | q.s (pH 10) | | |
| 1% w/w meglumine solution | | | | | 65% | 65% | | | | |
| 0.1 M triethanolamine solution | | | | | | | 65% | 65% | | |
| Carbonate buffer | | | | | | | | | 65% | 65% |
| 0.1 M $Na_2CO_3$ | | | | | | | | | q.s (pH 10.5) | q.s (pH 10) |
| 0.1 M $NaHCO_3$ | | | | | | | | | q.s (pH 10.5) | q.s (pH 10) |
| Deionised water | q.s | q.s | q.s | q.s | q.s | q.s | q.s | q.s | q.s | q.s |
| Total | 100% | 100% | 100% | 100% | 100% | 100% | 100% | 100% | 100% | 100% |
| Target pH | 10.5 | 10 | 10.5 | 10 | 10.5 | 10 | 10.5 | 10 | 10.5 | 10 |

Table 1: Compositions of the tested solutions

Table 2: Assay results of the tested solutions

| Average Recovery (%) | | | | |
|---|---|---|---|---|
| Tested solution | Time point / stored conditions | | | |
| | Initial | 7 days / 2-8 °C | 7 days / 25 °C | 7 days / 40 °C |
| Ia | 102.4 | 101.1 | 100.9 | 100.7 |
| Ib | 101.0 | 100.4 | 101.0 | 101.1 |
| IIa | 100.8 | 98.8 | 99.2 | 98.9 |
| IIb | 103.6 | 101.6 | 101.1 | 102.0 |
| IIIa | 104.1 | 101.7 | 100.2 | 104.2 |
| IIIb | 102.7 | 100.8 | 102.2 | 104.6 |
| IVa | 101.1 | 101.9 | 100.3 | 101.3 |
| IVb | 101.4 | 100.2 | 100.1 | 102.5 |
| Va | 103.7 | 101.9 | 101.9 | 101.8 |
| Vb | 101.6 | 102.3 | 100.2 | 101.8 |

The data from Table 2 indicates that:

- The assay results of all samples tested were 98.8 to 104.6% of the nominal concentration.
- There was no significant difference between T=0 and T=7 days assay data, this suggested that all samples remained stable over 7 days at different storage conditions. However, physical stability results indicate that formulations IIIa, IIIb (formulations of the invention, which include meglumine), Va, Vb (carbonate buffer) and IIa (Glycine-sodium hydroxide) are most physically stable under these storage conditions.

The data from Table 3 indicates that:

- ECA demonstrated pH dependent stability, the higher the pH the more stable the ECA solution was over 7 days at different storage conditions.
- Carbonate buffer, meglumine and glycine-sodium hydroxide respectively, showed API stability at all storage conditions at a pH above 9.4 for over 7 days.
- The assay results do not correlate with the physical stability of the samples, possible due to the samples dissolving in the diluents used for the analysis.

| | Appearance | | | | pH | | | |
|---|---|---|---|---|---|---|---|---|
| Tested solution | Time point / stored conditions | | | | Time point/stored condition | | | |
| | Initial | 7 days / 2-8 °C | 7 days / 25 °C | 7 days / 40 °C | Initial | 7 days / 2-8 °C | 7 days / 25 °C | 7 days / 40 °C |
| Ia | A clear pale yellow solution | A cloudy yellow solution | A cloudy yellow solution | A cloudy yellow solution | 10.0 | 8.9 | 9.2 | 9.2 |
| Ib | A very slightly pale yellow solution | A cloudy yellow solution (more cloudy than Ia) | A cloudy yellow solution (more cloudy than Ia) | A cloudy yellow solution | 9.0 | 9.0 | 9.0 | 9.0 |
| IIa | A clear pale yellow solution | A clear yellow solution | A clear yellow solution | A clear yellow solution | 10.5 | 9.4 | 9.1 | 9.5 |
| IIb | A clear pale yellow solution | A clear yellow solution | A clear yellow solution | A clear yellow solution | 10.1 | 10.0 | 10.0 | 10.0 |
| IIIa | A clear pale yellow solution | A clear yellow solution | A clear yellow solution | A clear yellow solution | 10.4 | 10.0 | 10.3 | 10.3 |
| IIIb | A clear pale yellow solution | A clear yellow solution | A clear yellow solution | A clear yellow solution | 9.9 | 10.0 | 10.1 | 10.0 |
| IVa | A clear pale yellow solution | A clear yellow solution | A clear yellow solution | A clear yellow solution | 10.4 | 9.3 | 9.7 | 9.8 |
| IVb | A clear pale yellow solution | A clear yellow solution | A clear yellow solution | A clear yellow solution | 9.9 | 9.5 | 9.4 | 9.3 |
| Va | A clear pale yellow solution | A clear yellow solution | A clear yellow solution | A clear yellow solution | 10.4 | 10.4 | 10.4 | 10.5 |
| Vb | A clear pale yellow solution | A clear yellow solution | A clear yellow solution | A clear yellow solution | 9.8 | 9.9 | 9.9 | 9.9 |

Table 3: Physical stability and pH results of the tested solutions

**Example 2 - formulations**

A series of formulations were prepared as follows.

- The required amount of ECA was dissolved in the required amount of propylene glycol at about 75°C.
- The ECA solution was mixed with the molten emulsifier at about 75°C and mixed until completely homogeneous.
- The required amount of pH modifiers was dissolved in the required amount of water (warmed up to 75°C) and then added to the ECA mixture at 75°C.
- For formulation D&E, the required amount of warmed buffer was added to the ECA mixture at about 75°C.
- The required amount of corn oil was added to the formulation at about 38°C followed by addition of all the remaining additives.
- The cream was mixed until completely homogeneous, and then allowed to stand overnight.

A generalisation of the compositions of formulations A to F are shown in Table 4.

Table 4: Generalisation of compositions of formulations A to F

| Ingredient | Composition of each formulation A to F (% w/w) | | | | | |
|---|---|---|---|---|---|---|
| | A | B | C | D | E | F |
| ECA | 1.00 | 1.00 | 1.00 | 1.00 | 1.00 | 1.00 |
| NaOH pellets | 0.18 | | | | | 0.18 |
| Carrier or vehicle | 56.16 | 56.16 | 56.16 | 56.16 | 56.16 | 56.16 |
| pH modifying agent | - | 1.00 | 1.50 | 41.95 | 41.95 | |
| Perfume | 0.89 | 0.89 | 0.89 | 0.89 | 0.89 | 0.89 |
| Deionised water | 41.77 | 40.95 | 40.45 | | | 41.77 |
| Total | 100% | 100% | 100% | 100% | 100% | 100% |

The formulations were tested for their stability over a 3 month period at 25°C and 40°C. The 3-month stability testing includes appearance, assay, viscosity and pH. The results of these tests are shown in Tables 5 to 8.

| | Appearance at each timepoint condition | | | | | | |
|---|---|---|---|---|---|---|---|
| **Formulation** | **T = 0** | **T = 14d 25 °C** | **T = 14d 40 °C** | **T = 28d 25 °C** | **T = 28d 40 °C** | **T = 3M 25 °C** | **T = 3M 40 °C** |
| A | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting |
| B | Off white, viscous cream with no sign of sediment or any splitting | Off white, viscous cream with no sign of sediment or any splitting | Off white, viscous cream with no sign of sediment or any splitting | Off white, viscous cream with no sign of sediment or any splitting | Off white, viscous cream with no sign of sediment or any splitting | Off white, viscous cream with no sign of sediment or any splitting | Off white, viscous cream with no sign of sediment or any splitting |
| C | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting |
| D | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting | White, viscous cream with no sign of sediment or any splitting |
| E | White, viscous cream with no sign of sediment or any splitting | White, viscous cream with no sign of sediment or any splitting | White, viscous cream with no sign of sediment or any splitting | White, viscous cream with no sign of sediment or any splitting | Off-White, viscous cream with no sign of sediment or any splitting | White, viscous cream with no sign of sediment or any splitting | Off-White, viscous cream with no sign of sediment or any splitting |
| F | White "lotion like" suspension with no sign of sediment or any splitting | White "lotion like" suspension with no sign of sediment or any splitting | White "lotion like" suspension with **obvious signs of splitting** | White "lotion like" suspension with no sign of sediment or any splitting | White "lotion like" suspension with **obvious signs of splitting** | White "lotion like" suspension with no sign of sediment or any splitting | White "lotion like" suspension with **obvious signs of splitting** |

Table 5: Appearance results for formulations A to F

| | Appearance at each timepoint condition | | | | | | | | |
|---|---|---|---|---|---|---|---|---|---|
| **Formulation** | **T = 0** | **T = 14d 25 °C** | **T = 14d 40 °C** | **T = 28d 25 °C** | **T = 28d 40 °C** | **T = 28d 40 °C** | **T = 28d 40 °C** | **T = 3M 25 °C** | **T = 3M 40 °C** |
| A | 105.4 | 108.3 | 107.7 | 106.8 | 97.8 | 97.8 | 97.8 | 105.8 | 94.5 |
| B | 106.8 | 108.6 | 107.5 | 105.7 | 108.7 | 110.7 | 106.8 | 106.7 | 100.8 |
| C | 107.2 | 108.2 | 106.7 | 106.8 | 106.4 | Not tested at this time point | | 108.1 | 103.2 |
| D | 107.0 | 106.0 | 109.4 | 107.7 | 97.8 | | | 106.0 | 95.4 |
| E | 106.2 | 106.5 | 105.3 | 105.7 | 103.2 | | | 105.5 | 101.7 |
| F | 105.7 | 107.8 | 105.1 | 106.3 | 99.9 | | | 105.1 | 98.7 |

Table 6: Assay results for formulations A to F

| Formulation | Appearance at each timepoint condition | | | | | | |
|---|---|---|---|---|---|---|---|
| | T = 0 | T = 14d 25 °C | T = 14d 40 °C | T = 28d 25 °C | T = 28d 40 °C | T = 3M 25 °C | T = 3M 40 °C |
| A | 159400 cP (%RSD = 3.6) | 229200 cP (%RSD = 3.4) | 229200 cP (%RSD = 6.3) | 243100 cP (%RSD = 6.4) | 219900 cP (%RSD = 5.7) | 242900 cP (%RSD= 5.5) | 212700 cP (%RSD = 4.8) |
| B | 139200 cP (%RSD = 1.9) | 178700 cP (%RSD = 3.4) | 188000 cP (%RSD = 6.9) | 167800 cP (%RSD= 22.1) | 151600 cP (%RSD = 9.7) | 201900 cP (%RSD= 4.5) | 156200 cP (%RSD = 5.0) |
| C | 300800 cP (%RSD= 4.1) | 300800 cP (%RSD = 5.1) | 310600 cP (%RSD = 5.7) | 266900 cP (%RSD = 34.1) | 274500 cP (%RSD = 7.7) | 313100 cP (%RSD= 2.7) | 213200 cP (%RSD = 6.4) |
| D | 155900 cP (%RSD= 9.0) | 230400 cP (%RSD = 3.4) | 180300 cP (%RSD = 7.4) | 243900 cP (%RSD = 9.0) | 184800 cP (%RSD=23.1) | 235700 cP (%RSD= 4.9) | 165900 cP (%RSD = 4.8) |
| E | 175400 cP (%RSD= 2.6) | 235700 cP (%RSD = 4.4) | 205600 cP (%RSD = 3.7) | 241200 cP (%RSD = 5.1) | 163300 cP (%RSD = 5.0) | 226500 cP (%RSD= 3.1) | 138900 cP (%RSD = 2.2) |
| F | 27600 cP (%RSD= 2.5) | 35500 cP (%RSD = 4.0) | Not tested due to sample appearance | 23200 cP (%RSD = 3.4 | Not tested due to sample appearance | 20100 cP (%RSD= 4.9) | Not tested due to sample appearance |

Table 7: Viscosity results for formulations A to F

Table 8: pH results for formulations A to F

| Appearance at each timepoint condition | | | | | | | |
|---|---|---|---|---|---|---|---|
| **Formulation** | **T = 0** | **T = 14d 25 °C** | **T=14d 40 °C** | **T = 28d 25 °C** | **T = 28d 40 °C** | **T=3M 25 °C** | **T = 3M 40 °C** |
| A | 9.83 | 9.34 | 9.56 | 9.70 | 9.64 | 9.54 | 9.44 |
| B | 9.33 | 9.34 | 9.38 | 9.64 | 9.38 | 9.47 | 9.09 |
| C | 7.70 | 8.61 | 8.60 | 8.70 | 8.61 | 8.50 | 8.40 |
| D | 8.91 | 9.24 | 9.46 | 9.27 | 9.16 | 9.04 | 9.04 |
| E | 8.85 | 9.37 | 9.33 | 9.53 | 9.45 | 9.35 | 9.13 |
| F | 9.74 | 9.63 | 9.15 | 9.58 | 9.29 | 9.69 | 9.22 |

The data from Table 5 indicates that:

- Formulation A to D are physically stable for 3 months at both 25°C and 40°C storage conditions.
- Formulation E is physically stable for 3 months at 25°C storage condition but shows a change in appearance at 40°C for 28 days and 3 months (the colour of the cream changed from white to off white).
- Formulation F is physically stable for 3 months at 25°C but unstable at 40°C within 14 days as signs of the cream splitting was noted.

The data from Table 6 indicates that:

- The T=0 assay of all formulations is slightly high; this is possibly due to water lost during processing at high temperature.
- There was no significant difference between T=0 and T=28 day (at both 25°C and 40°C) assay data for formulation B, C and E.
- Formulation A, D and F demonstrate no significant change in assay for 28 days at only 25°C storage but there is a drop at 40°C.
- There were no significant changes from T=0 to T=2 month assay results of formulation B at both storage conditions, whereas the assay of formulation A has dropped following storage at 40°C for 2 months.
- There is little or no change in assay results all formulations at 3 months upon storage at the lower storage temperature (25°C). however, there was a drop in assay of all prototypes at 3 months storage at the higher storage temperature (40°C) but the assay results of formulations B, formulation C and formulation E were still more than 100% upon storage at 40°C for 3 months.
- Formulation E appears to have a relatively stable assay over the 3-month period at 25°C and demonstrates the least variability in the assay at 40°C.

The data from Table 7 indicates that:

- Following 3 months of storage, the formulations demonstrated a temperature dependent trend with the samples stored at 40°C showing slightly lower viscosity as compared to the same sample stored at 25°C. This is as expected since the viscosity of the sample is inversely proportional to the temperature.

The data from Table 8 indicates that:

- The T=0 pH measurements of the formulations are similar to the marketed product except formulation C which has slightly lower pH due to the amount of ethanolamine in the formulation, with no additional pH adjustment made during processing.
- There was a slight drop in pH of all prototypes at 3 months upon storage at 40°C, however there was no significant change in the pH of any of the formulations following storage at 25°C for 3 months.

**Example 3 - optimization of formulations**

All formulations were manufactured at a 350 g scale except formulation B1 which was manufactured at a scale of 1kg.

A preparation summary based on the "A" formulation type is as follows:

- The NaOH pellets were dissolved into deionised water.
- Sodium, cetostearyl sulphate was then added to the NaOH solution and with heating, this dissolved and formed a slightly viscous solution.
- Propylene glycol was warmed and the required amount of ECA was dissolved in the required amount of propylene glycol at about 75°C.
- The propylene glycol-ECA mixture was then added to warmed diethylene glycol monostearate.
- The NaOH solution was then added warm to the ECA mixture at 75°C.
- The required amount of corn oil was added to the formulation at about 38°C followed by addition of all the remaining additives as per Table 2.
- The cream was mixed until completely homogeneous, and then allowed to stand overnight.

A preparation summary based on the "B" formulations is as follows:

- For B2 and B4, Sodium cetostearyl sulphate was then added to deionised water and with heating.
- For B1 and B3, deionised water was heated.
- For B2 and B4, Meglumine was added to the sodium cetostearyl sulphate solution.
- For B1 and B3, meglumine was added to the deionised water.
- Propylene glycol was warmed and the required amount of ECA was dissolved in the required amount of propylene glycol at about 75°C.
- The propylene glycol-ECA mixture was then added to warmed diethylene glycol monostearate.
- The solutions were then combined at 75°C and mixed with a paddle stirrer.
- The required amount of corn oil was added to the formulation at about 38°C followed by addition of all the remaining additives as per Table 2.
- The cream was mixed until completely homogeneous, and then allowed to stand overnight.

The general compositions of the different formulations are shown in Table 9.

Compositions A1 and A2 are provided for reference purposes only.

Table 9: Compositions for formulations A1, A2 and B1 to B4.

| Ingredient | Range (% w/w) | Composition | | | | | |
|---|---|---|---|---|---|---|---|
| | | A1 | B1 | B2 | B3 | B4 | A2 |
| ECA | 0.8 - 1.2% | x | x | x | x | x | x |
| NaOH pellets | 0.15-0.20 | x | | | | | x |
| Diethylene glycol monostearate | 15 - 25% | x | x | x | x | x | x |
| Propylene glycol | 25 - 35% | x | x | x | x | x | x |
| Corn oil | 5 - 15 % | x | x | x | x | x | x |
| Perfume | 0.8 - 1.2% | x | x | x | x | x | x |
| Deionised water | 25 - 50% | x | x | x | x | x | x |
| Meglumine | 0.5 - 1.2% | | x | x | x | x | |
| Sodium cetostearyl sulphate | 0.35 - 1.2 | x | | x | | x | x |
| Total | 100 | 100 | 100 | 100 | 100 | 100 | 100 |

The formulations were subjected to stability testing for periods of 1 month and 3 months at temperatures of 25°C and 40°C. They were analysed in terms of their appearance, viscosity, pH and assay.

Appearance testing performed on all formulations indicated that there was no change in appearance at both 25°C and 40°C storage conditions at the 3 month timepoint. The appearance of all formulations were recorded as a white viscous cream with no sign of sediment or splitting. The results of the viscosity, pH and assay testing are shown below in Tables 10 to 12.

Table 10: viscosity test results for A1, A2 and B1 to B4.

| Sample | Viscosity (cP) | | | | |
|---|---|---|---|---|---|
| | 0 days | 28 days | | 3 months | |
| | 25 °C | 25 °C mean | 40 °C mean | 25 °C mean | 40 °C mean |
| A1 | 247700 | 347400 | 284200 | 304400 | 229900 |
| B1 | 272900 | 336100 | 305500 | 255300 | 324500 |
| B2 | 294900 | 330000 | 240100 | 304300 | 244500 |
| B3 | 272400 | 301200 | 288100 | 270900 | 216400 |
| B4 | 218000 | 207600 | 220900 | 204800 | 216800 |
| A2 | 267600 | 329900 | 305500 | 374400 | 274700 |

Table 11: pH test results for A1, A2 and B1 to B4.

| Sample | pH | | | | |
|---|---|---|---|---|---|
| | 0 days | 28 days | | 3 months | |
| | 25 °C | 25 °C mean | 40 °C mean | 25 °C mean | 40 °C mean |
| A1 | 9.77 | 9.63 | 9.76 | 9.12 | 8.78 |
| B1 | 9.63 | 9.74 | 9.55 | 9.34 | 9.07 |
| B2 | 9.61 | 9.73 | 9.55 | 9.30 | 8.86 |
| B3 | 9.84 | 9.96 | 9.75 | 9.54 | 9.21 |
| B4 | 9.58 | 9.75 | 9.43 | 9.24 | 8.68 |
| A2 | 9.73 | 9.85 | 9.51 | 9.37 | 8.68 |

Table 12: assay results for A1, A2 and B1 to B4.

| Sample | Mean assay value (%) | | | | | | |
|---|---|---|---|---|---|---|---|
| | 25 °C timepoints (Days) | | | Change in mean assay at 25 °C from T = 0 (%) | 40 °C timepoints (Days) | | Change in mean assay at 40°C from T = 0 (%) |
| | 0 | 28 | 84 | | 28 | 84 | |
| A1 | 107.0 | 107.0 | 104.5 | -2.5 | 103.6 | 98.8 | -8.2 |
| B1 | 107.7 | 108.2 | 104.5 | -3.2 | 107.2 | 102.7 | -5.0 |
| B2 | 106.1 | 106.4 | 103.0 | -3.1 | 106.4 | 99.7 | -6.4 |
| B3 | 104.6 | 105.4 | 103.4 | -1.2 | 106.0 | 104.0 | -0.6 |
| B4 | 107.4 | 106.2 | 105.2 | -2.2 | 105.9 | 100.2 | -7.2 |
| A2 | 102.1 | 107.0 | 100.8 | -1.3 | 102.0 | 83.9 | -18.2 |

The viscosity results demonstrate a lowering in viscosity for the samples stored at 40°C compared to those stored at 25°C. This is expected due to the increase in storage temperature.

The initial pH measurement of all formulations were within the range of 9.6 to 9.8.

Following 28 days storage there was an observed drop in the pH of formulation A2 and B4 upon storage at 40°C, however, the pH values of these formulations remained higher than pH 9.3. Analysis of 3 months stability samples indicated a greater decrease in pH at both 25°C and 40°C with formulations A1 and B2 dropping below pH 9.3. Formulation B2 was observed to maintain the highest pH with a pH of 9.54 observed at 25°C and a pH of 9.21 at 40°C. Additionally, formulation B1 was observed to decrease at a similar rate as formulation B1. For formulations A1, A2, B2 and B4 a much greater decrease in pH was observed. This difference in rate of decrease suggests formulations B1 and B3 are more favourable.

Formulation A1 demonstrated a slight drop in assay at the 40°C temperature, at 28 days. Further analysis at the 3

months timepoint indicated a notable decrease in the assay of both formulation A1 and A2 stored at 40°C of 8.2% and 18.2% respectively. Although a notable decrease in assay was observed within these formulations a large variance was also observed between sample replicates. This is possibly due to the preparation of replicates from two separate vials and may indicate a difference in either formulation homogeneity or degradation between sample vials. For formulations B1, B2 and B4 a slower decrease in assay was observed at 40°C of 5.0%, 6.4% and 7.2% respectively. For formulation B3 the decrease in assay at 40°C was notably reduced as compared to other formulations with a decrease of 0.6% observed. This suggests the assay of formulation B3 is more stable than the other formulations and further supports the increased B3 stability observed for pH results.

## Claims

**1.** A pharmaceutical composition suitable for topical application which comprises *Centella Asiatica* extract (ECA) and a pH modifying agent which is meglumine, and wherein said composition has a pH from 9.0 to 10.5.

**2.** A pharmaceutical composition according to claim 1, wherein the composition comprises from 0.25 to 5.0 wt% ECA.

**3.** A pharmaceutical composition according to claim 1 or claim 2, wherein the composition comprises from 0.5 to 2.5 wt% ECA.

**4.** A pharmaceutical composition according to any one of the preceding claims, wherein the composition comprises a carrier or vehicle selected from water, petroleum hydrocarbons, fatty acids, fatty acid esters, fatty alcohols, fatty alcohol ethers, fatty alcohol esters, $C_2$-$C_8$ linear or branched, saturated or unsaturated alcohols, polyols, aromatic alcohols, alkyleneglycol ethers, alkyleneglycol esters, natural waxes, silicones or mixtures thereof.

**5.** A composition according to claim 4, wherein the carrier or vehicle is selected from water, fatty acid esters, polyols or mixtures thereof.

**6.** A composition according to any one of the preceding claims, wherein the composition further comprises one or more additives selected from anti-irritants agents, antioxidants agents, chelating agents, emollients, penetration enhancing agents, preservative agents, solubilizing agents, thickening agents, wetting agents, fragrances or mixtures thereof.

**7.** A composition according to any one of the preceding claims, wherein the composition is formulated in the form of a cream, a gel, an ointment, a paste, a suspension, a lotion, a foam, a spray, an aerosol or a solution.

**8.** A composition according to any one of the preceding claims, wherein the composition is formulated in the form of an ointment.

**9.** A composition according to any one of the preceding claims, which comprises:

(a) from 0.25 to 5.0 wt% ECA
(b) from 0.2 to 2.5 wt% meglumine
(c) from 0.5 to 2.5 wt% additives
(d) from 90 to 98.8 wt % carrier or vehicle.

**10.** A composition according to any one of the preceding claims, which comprises:

(a) from 0.8 to 1.2 wt% ECA
(b) from 0.5 to 2 wt% meglumine
(c) from 0.8 to 1.2 wt% of a perfume or mixture of perfumes
(d) from 15 to 25 wt % diethylene glycol monostearate
(e) from 25 to 35 wt% propylene glycol
(f) from 5 to 15 wt% corn oil
(g) from 25 to 50 wt% water.

**11.** A composition as defined in any one of claims 1 to 10, for use in the treatment of wounds.

**12.** The composition for use as defined in claim 11, wherein the wounds are fissures, ulcers, sores, pressure ulcers or

burns.

**Patentansprüche**

**1.** Pharmazeutische Zusammensetzung, die zur topischen Anwendung geeignet ist und *Centella-Asiatica*-Extrakt (ECA) sowie ein pH-Modifizierungsmittel, das Meglumin ist, umfasst, und wobei die Zusammensetzung einen pH-Wert von 9,0 bis 10,5 aufweist.

**2.** Pharmazeutische Zusammensetzung nach Anspruch 1, wobei die Zusammensetzung 0,25 bis 5,0 Gew.-% ECA umfasst.

**3.** Pharmazeutische Zusammensetzung nach Anspruch 1 oder Anspruch 2, wobei die Zusammensetzung 0,5 bis 2,5 Gew.-% ECA umfasst.

**4.** Pharmazeutische Zusammensetzung nach einem der vorstehenden Ansprüche, wobei die Zusammensetzung einen Träger oder ein Vehikel umfasst, ausgewählt aus Wasser, Erdölkohlenwasserstoffen, Fettsäuren, Fettsäureestern, Fettalkoholen, Fettalkoholethern, Fettalkoholestern, linearen oder verzweigten, gesättigten oder ungesättigten $C_2$-$C_8$-Alkoholen, Polyolen, aromatischen Alkoholen, Alkylenglykolethern, Alkylenglykolestern, natürlichen Wachsen, Silikonen oder Mischungen davon.

**5.** Zusammensetzung nach Anspruch 4, wobei der Träger oder das Vehikel ausgewählt ist aus Wasser, Fettsäureestern, Polyolen oder Mischungen davon.

**6.** Zusammensetzung nach einem der vorstehenden Ansprüche, wobei die Zusammensetzung ferner ein oder mehrere Additive umfasst, ausgewählt aus reizlindernden Mitteln, Antioxidantien, Chelatbildnern, Weichmachern, Penetrationsverstärkern, Konservierungsmitteln, Lösungsvermittlern, Verdickungsmitteln, Netzmitteln, Duftstoffen oder Mischungen davon.

**7.** Zusammensetzung nach einem der vorstehenden Ansprüche, wobei die Zusammensetzung in Form einer Creme, eines Gels, einer Salbe, einer Paste, einer Suspension, einer Lotion, eines Schaums, eines Sprays, eines Aerosols oder einer Lösung formuliert ist.

**8.** Zusammensetzung nach einem der vorstehenden Ansprüche, wobei die Zusammensetzung in Form einer Salbe formuliert ist.

**9.** Zusammensetzung nach einem der vorstehenden Ansprüche, die Folgendes umfasst:

(a) 0,25 bis 5,0 Gew.-% ECA
(b) 0,2 bis 2,5 Gew.-% Meglumin
(c) 0,5 bis 2,5 Gew.-% Additive
(d) 90 bis 98,8 Gew.-% Träger oder Vehikel.

**10.** Zusammensetzung nach einem der vorstehenden Ansprüche, die Folgendes umfasst:

(a) 0,8 bis 1,2 Gew.-% ECA
(b) 0,5 bis 2 Gew.-% Meglumin
(c) 0,8 bis 1,2 Gew.-% eines Duftstoffs oder einer Duftstoffmischung
(d) 15 bis 25 Gew.-% Diethylenglykolmonostearat
(e) 25 bis 35 Gew.-% Propylenglykol
(f) 5 bis 15 Gew.-% Maisöl
(g) 25 bis 50 Gew.-% Wasser.

**11.** Zusammensetzung, wie definiert in einem der Ansprüche 1 bis 10, zur Verwendung bei der Behandlung von Wunden.

**12.** Zusammensetzung zur Verwendung wie in Anspruch 11 definiert, wobei es sich bei den Wunden um Risse, Geschwüre, Wundstellen, Druckgeschwüre oder Verbrennungen handelt.

## Revendications

1. Composition pharmaceutique adaptée à une application topique, comprenant un extrait de *Centella asiatica* (ECA) et un agent modificateur de pH, à savoir la méglumine, ladite composition présentant un pH de 9,0 à 10,5.

2. Composition pharmaceutique selon la revendication 1, ladite composition comprenant de 0,25 à 5,0 % en poids d'ECA.

3. Composition pharmaceutique selon la revendication 1 ou la revendication 2, ladite composition comprenant de 0,5 à 2,5 % en poids d'ECA.

4. Composition pharmaceutique selon l'une quelconque des revendications précédentes, ladite composition comprenant un excipient ou un vecteur choisi parmi l'eau, les hydrocarbures pétroliers, les acides gras, les esters d'acides gras, les alcools gras, les éthers d'alcools gras, les esters d'alcools gras, les alcools en $C_2$-$C_8$ linéaires ou ramifiés, saturés ou insaturés, les polyols, les alcools aromatiques, les éthers d'alkylèneglycol, les esters d'alkylèneglycol, les cires naturelles, les silicones ou les mélanges de ceux-ci.

5. Composition selon la revendication 4, dans laquelle l'excipient ou le vecteur est choisi parmi l'eau, les esters d'acides gras, les polyols ou des mélanges de ceux-ci

6. Composition selon l'une quelconque des revendications précédentes, ladite composition comprenant en outre un ou plusieurs additifs choisis parmi les agents anti-irritants, les agents antioxydants, les agents chélateurs, les émollients, les agents favorisant la pénétration, les agents conservateurs, les agents solubilisants, les agents épaississants, les agents mouillants, les parfums ou les mélanges de ceux-ci

7. Composition selon l'une quelconque des revendications précédentes, ladite composition étant formulée sous la forme d'une crème, d'un gel, d'une pommade, d'une pâte, d'une suspension, d'une lotion, d'une mousse, d'un spray, d'un aérosol ou d'une solution.

8. Composition selon l'une quelconque des revendications précédentes, ladite composition étant formulée sous la forme d'une pommade.

9. Composition selon l'une quelconque des revendications précédentes, comprenant :

   (a) de 0,25 à 5,0 % en poids d'ECA
   (b) de 0,2 à 2,5 % en poids de méglumine
   (c) de 0,5 à 2,5 % en poids d'additifs
   (d) de 90 à 98,8 % en poids d'excipient ou de vecteur.

10. Composition selon l'une quelconque des revendications précédentes, comprenant :

    (a) de 0,8 à 1,2 % en poids d'ECA
    (b) de 0,5 à 2 % en poids de méglumine
    (c) de 0,8 à 1,2 % en poids d'un parfum ou d'un mélange de parfums
    (d) de 15 à 25 % en poids de monostéarate de diéthylène glycol
    (e) de 25 à 35 % en poids de propylène glycol
    (f) de 5 à 15 % en poids d'huile de maïs
    (g) de 25 à 50 % en poids d'eau.

11. Composition telle que définie dans l'une quelconque des revendications 1 à 10, destinée à être utilisée dans le traitement des plaies.

12. Composition destinée à une utilisation telle que définie dans la revendication 11, dans laquelle les plaies sont des fissures, des ulcères, des lésions, des escarres ou des brûlures.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105902476
- WO 2016181342 A
- CN 111374941
- CN 108567638
- WO 2021010953 A
- US 2019282538 A
- WO 2018221487 A
- JP 2010143868 A